(19) Europäisches Patentamt
European Patent Office
Office européen des brevets

(11) Veröffentlichungsnummer: **0 006 978**
**B1**

(12) # EUROPÄISCHE PATENTSCHRIFT

(45) Veröffentlichungstag der Patentschrift: **29.01.86**

(21) Anmeldenummer: **79101242.0**

(22) Anmeldetag: **25.04.79**

(51) Int. Cl.⁴: **C 07 C 69/73,** C 07 C 69/743, C 07 C 121/75, C 07 C 149/415, C 07 D 317/46, C 07 D 319/20, A 01 N 53/00, C 07 C 67/14, C 07 C 67/10, C 07 C 43/23, C 07 C 41/26

(54) **Benzylester mit fluorsubstituierten Äther- und/oder Thioäthergruppen, ihre Herstellungen und ihre Verwendung als Insektizide; Zwischenprodukte und ihre Herstellungen.**

(30) Priorität: **05.05.78 DE 2819788**

(43) Veröffentlichungstag der Anmeldung: **23.01.80 Patentblatt 80/2**

(45) Bekanntmachung des Hinweises auf die Patenterteilung: **29.01.86 Patentblatt 86/5**

(84) Benannte Vertragsstaaten: **BE CH DE FR GB IT NL**

(56) Entgegenhaltungen:
**DE - A - 2 333 849**
**DE - A - 2 428 372**
**DE - A - 2 757 066**
**DE - A - 2 808 627**
**FR - A - 2 271 196**
**US - A - 3 666 789**
**US - A - 4 002 628**

**C.A. 70, 28544z**
**J.Med.Chem., 16, 1400-1**
**Bull.Soc.Chim.Fr. (1961) 325-9**
**Bull.Soc.Chim.Fr. (1957) 921-3**
**I.O.C. 29, 1-11**
**I.O.C. 37, 673**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen (DE)**
Erfinder: **Marhold, Albrecht, Dr., Carl-Duisberg-Strasse 329, D-5090 Leverkusen (DE)**
Erfinder: **Behrenz, Wolfgang, Dr., Untergruendemich 14, D-5063 Overath (DE)**
Erfinder: **Hammann, Ingeborg, Dr., Belfortstrasse 9, D-5000 Köln (DE)**

**EP 0 006 978 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue Benzylester mit fluorsubstituierten Äther- und/oder Thioäthergruppen, Verfahren zu ihrer Herstellung, ihre Verwendung als Insektizide sowie neue Zwischenprodukte zur Herstellung dieser Wirkstoffe.

Ähnliche Wirkstoffe sind bereits bekannt aus der franz. Patentschrift 2 290 415.

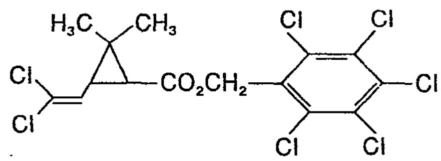

oder liegen im Handelsprodukt Neopynamin®

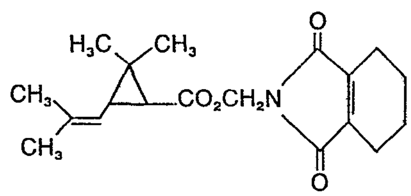

vor. Diese Verbindungen besitzen jedoch den Nachteil zu geringer Wirksamkeit vor allem bei niedrigen Aufwandkonzentrationen.

1. Es wurden die neuen Benzylester der Formel I gefunden

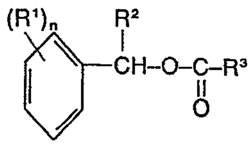 (I)

in welcher

n für 1–5 steht und

$R^1$ für gleiche oder verschiedene Reste aus der Gruppe Wasserstoff, $C_{1-6}$-Alkyl, $C_{1-6}$-Halogenalkyl mit 3–6 Halogenatomen, $C_{1-6}$-Alkoxy, $C_{1-6}$-Halogenalkoxy mit 1–6-Halogenatomen, $C_{1-6}$-Alkylmercapto, $C_{1-6}$-Halogenalkylmercapto mit 1–6 Halogenatomen, Halogen, gegebenenfalls substituiertes Phenyl, gegebenenfalls substituiertes Phenoxy, steht oder zwei benachbarte Reste gemeinsam mit den angrenzenden C-Atomen einen gegebenenfalls substituierten ankondensierten Benzolring oder ein oder mehrfach durch Fluor substituierte Sauerstoff enthaltende heterocyclische Fünf- oder Sechsringe bilden, wobei in Formel I entweder einer der Reste $R^1$ für Fluoralkoxy oder Fluoralkylmercapto stehen muss oder wobei zwei benachbarte Reste gemeinsam mit den angrenzenden C-Atomen ein oder mehrfach durch Fluor substituierte Sauerstoff enthaltende heterocyclische Fünf- oder Sechsringe bilden müssen,

$R^2$ für Wasserstoff, $C_{1-4}$-Alkyl, Cyan oder Ethinyl steht,

$R^3$ für Reste der Formel

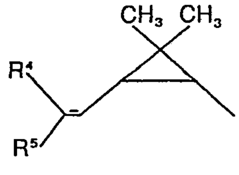 oder 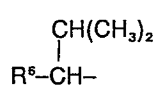

$R^4$ und $R^5$ für Chlor oder Brom stehen,

$R^6$ für gegebenenfalls durch Halogen, Alkyl, Alkylthio, Alkoxy mit jeweils 1–4 C-Atomen, Nitro, Methylendioxy substituiertes Phenyl steht.

2. Es wurde ferner gefunden, dass man die neuen Benzylester der Formel I erhält, indem man

a) Carbonylhalogenide der Formel II

$$Hal–CO–R^3 \qquad (II)$$

in welcher

$R^3$ die (oben) angegebene Bedeutung hat, und

Hal für Halogen, vorzugsweise Chlor, steht,

mit Benzylalkoholen der Formel III

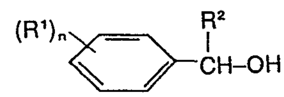 (III)

in welcher

$R^1$, n und $R^2$ die oben angegebene Bedeutung haben,

gegebenenfalls in Gegenwart eines Säureakzeptors und gegebenenfalls in Gegenwart eines Lösungsmittels umsetzt, oder

b) Carbonsäuresalze der Formel IV

$$MeO–CO–R^3 \qquad (IV)$$

in welcher

Me K oder Na bedeutet und

$R^3$ die oben angegebene Bedeutung hat,

mit Benzylhalogeniden der Formel V

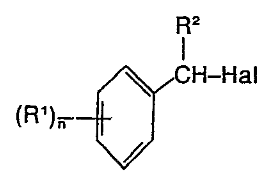 (V)

in welcher

$R^1$, n und $R^2$ die oben angegebene Bedeutung haben und

Hal Chlor oder Brom bedeutet,

gegebenenfalls in Gegenwart eines Lösungsmittels und gegebenenfalls in Gegenwart eines quartären Ammoniumsalzes umsetzt.

3. Es wurden ferner die neuen Benzylalkohole der Formel IIIa gefunden

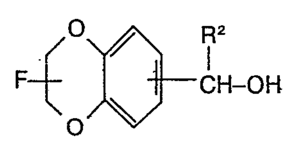 (IIIa)

in welcher

$R^2$ die unter 1 (oben) angegebene Bedeutung hat und wobei die übrigen Stellen des Benzolringes durch Halogen substituiert sein können.

4. Es wurde ferner gefunden, dass man die neuen Benzylalkohole der Formel IIIa gemäss 3 (oben) erhält, indem man

a) Aldehyde der Formel VIa

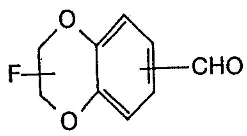 (VIa)

in welcher

die übrigen Stellen des Benzolrings durch Halogen substituiert sein können,

für den Fall, dass $R^2$ im Benzylalkohol für Wasserstoff steht, reduziert, oder für den Fall, dass $R^2$ im Benzylalkohol für CN steht, mit HCN umsetzt,

oder für den Fall, dass $R^2$ im Benzylalkohol für $C_{1-4}$-Alkyl oder Ethinyl steht, mit einer Grignard-Verbindung der Formel VII

$R^2$-Mg-Hal (VII)

in welcher

$R^2$ für $C_{1-4}$-Alkyl oder Ethinyl steht,

umsetzt, oder

b) dass man Benzylamine der Formel VIIIa

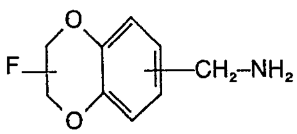 (VIIIa)

in welcher

die übrigen Stellen des Benzolrings durch Halogen substituiert sein können,

mit Natrium- oder Kaliumnitrit in Gegenwart einer Säure umsetzt oder

c) dass man ein Benzylhalogenid der Formel Va

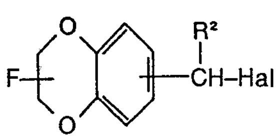 (Va)

in welcher

$R^2$ die unter 1 (oben) angegebene Bedeutung besitzt,

Hal für Halogen steht und die übrigen Stellen des Benzolrings durch Halogen substituiert sein können, mit wässrigen Basen verseift.

5. Es wurden ferner die neuen Benzylhalogenide der Formel Va gefunden

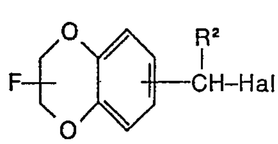 (Va)

in welcher

$R^2$ die unter 1 (oben) angegebene Bedeutung besitzt, Hal für Halogen steht und die übrigen Stellen des Benzolrings durch Halogen substituiert sein können.

6. Es wurde ferner gefunden, dass man die neuen Benzylhalogenide der Formel V gemäss 5 (oben) erhält, indem man Verbindungen der Formel IXa

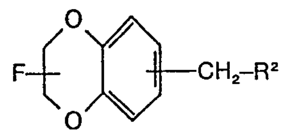 (IXa)

in welcher

$R^2$ die unter 1 (oben) angegebene Bedeutung hat und die übrigen Stellen des Benzolrings durch Halogen substituiert sein können,

in der Seitenkette auf an sich bekannte Weise halogeniert.

7. Es wurden ferner die neuen Aldehyde der Formel VIa gefunden

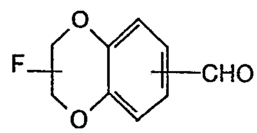 (VIa)

in welcher die übrigen Stellen des Benzolrings durch Halogen substituiert sein können.

8. Es wurde ferner gefunden, dass man die neuen Aldehyde der Formel VIa gemäss 7 (oben) erhält, indem man in Verbindungen der Formel XIa

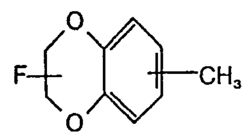 (XIa)

in welcher

die übrigen Stellen des Benzolrings durch Halogen substituiert sein können,

die $CH_3$-Gruppe in an sich bekannter Weise zur $-CH\ Hal_2$-Gruppe halogeniert und anschliessend in üblicher Weise zum Aldehyd der Formel VIa verseift.

9. Es wurden ferner die neuen Benzylamine der Formel VIIIa gefunden,

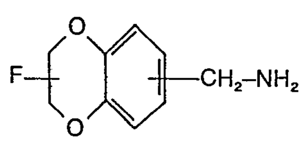 (VIIIa)

in welcher

die übrigen Stellen des Benzolrings durch Halogen substituiert sein können.

10. Es wurde ferner gefunden, dass man die Benzylamine der Formel VIIIa gemäss 9 (oben) erhält, indem man eine Verbindung der Formel XIIa

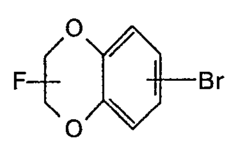 (XIIa)

in welcher

die übrigen Stellen des Benzolrings durch Halogen substituiert sein können,

in einer ersten Stufe mit einem Cyanidsalz umsetzt und das dabei erhaltene Nitril der Formel XIIIa

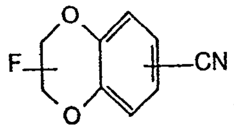

(XIIIa)

in welcher

die übrigen Stellen des Benzolrings durch Halogen substituiert sein können,

in an sich bekannter Weise nitriert.

Die Verbindungen der Formel I gemäss 1 (oben) zeigen gute insektizide Eigenschaften.

Überraschenderweise zeigen die neuen erfindungsgemässen Wirkstoffe gemäss 1 (oben) eine erheblich höhere Wirksamkeit als die aus dem Stand der Technik bekannten Verbindungen.

Von den erfindungsgemässen Verbindungen der Formel I gemäss 1 (oben) sind bevorzugt diejenigen, in denen

n für 1–5 steht, und

$R^1$ für gleiche oder verschiedene Reste aus der Gruppe Wasserstoff, Fluor, Chlor, Brom, Trifluormethyl, Methoxy, Difluormethoxy, Trifluormethoxy, Tetrafluoräthoxy, Hexafluorpropoxy, Difluormethylthio, Trifluormethylthio oder für Phenyl oder Phenoxy, die gegebenenfalls durch Halogen oder Alkoxy substituiert sind, stehen oder zwei benachbarte Reste gemeinsam mit den angrenzenden C-Atomen einen mehrfach durch Fluor substituierten Sauerstoff enthaltenden Fünf- oder Sechsring bilden, wobei mindestens einer der Reste Fluoralkoxy oder Fluormethylthio bedeuten soll, oder zwei der Reste gemeinsam mit den angrenzenden C-Atomen einen heterocyclischen Fünf- oder Sechsring bilden und für $OCF_2O$, $OCF_2CH_2O$ oder $OCF_2CHFO$ stehen,

$R^2$ für Wasserstoff, $C_1$–$C_4$-Alkyl-, Cyan oder Äthinyl steht und

$R^3$ für den Rest

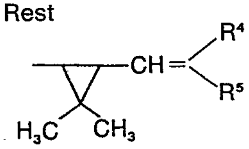

steht, wobei

$R^4$ und $R^5$ gleich oder verschieden sind und für Chlor oder Brom

stehen, oder $R^3$ für den Rest

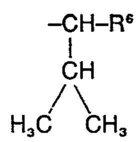

steht, wobei

$R^6$ für einen gegebenenfalls durch Halogen, Alkyl, Alkylthio, Alkoxy mit jeweils 1–4 C-Atomen, Nitro, Methylendioxy substituierten Phenylring steht.

Ganz besonders bevorzugt sind solche Verbindungen der Formel I in welcher $R^1$ für gleiche oder verschiedene Reste der Gruppe Wasserstoff, Chlor, Difluormethoxy, Tetrafluoräthoxy, Hexafluorpropoxy oder Trifluormethylthio steht oder zwei benachbarte Reste $R^1$ gemeinsam mit den angrenzenden C-Atomen einen heterocyclischen Sauerstoff enthaltenden 5- oder 6-Ring bilden, wobei mindestens ein Rest Difluormethoxy, Tetrafluoräthoxy, Hexafluorpropoxy oder Trifluormethylthio bedeuten soll, oder zwei benachbarte Reste gemeinsam mit den angrenzenden C-Atomen einen heterocyclischen 5- oder 6-Ring bilden und für $OCF_2O$, $OCF_2$–$CH_2O$ oder $OCF_2CHFO$ stehen, $R^2$ für Wasserstoff oder Cyan steht und $R^3$ für den Rest

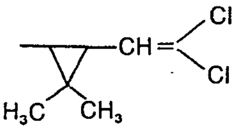

oder für den Rest

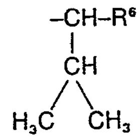

wobei $R^6$ für einen durch Fluor, Chlor oder Brom, Methoxy oder Methylendioxy substituierten Phenylring steht.

Folgende Verbindungen der Formel I seien im einzelnen genannt:

2-Difluormethoxybenzyl-2,2-dimethyl-3-(2′,2′-dichlorvinyl)-cyclopropancarboxylat,

3-Difluormethoxybenzyl-2,2-dimethyl-3-(2′,2′-dichlorvinyl)-cyclopropancarboxylat,

2-Tetrafluoräthoxybenzyl-2,2-dimethyl-3-(2′,2′-dichlorvinyl)-cyclopropancarboxylat,

3-Tetrafluoräthoxybenzyl-2,2-dimethyl-3-(2′,2′-dichlorvinyl)-cyclopropancarboxylat,

3-Hexafluorpropoxybenzyl-2,2-dimethyl-3-(2′,2′-dichlorvinyl)-cyclopropancarboxylat,

3-Difluormethoxy-4-chlorbenzyl-2,2-dimethyl-3-(2′,2′-dichlorvinyl)-cyclopropancarboxylat,

3,4-Bis-(difluormethoxy)-benzyl-2,2-dimethyl-3-(2′,2′-dichlorvinyl)-cyclopropancarboxylat,

3-Trifluormethylthio-benzyl-2,2-dimethyl-3-(2′,2′-dichlorvinyl)-cyclopropancarboxylat,

3-Trifluormethoxy-benzyl-2,2-dimethyl-3-(2′,2′-dichlorvinyl)-cyclopropancarboxylat,

3-Difluormethylthio-benzyl-2,2-dimethyl-3-(2′,2′-dichlorvinyl)-cyclopropancarboxylat,

3-Difluormethoxy-α-cyano-benzyl-2,2-dimethyl-3-(2′,2′-dichorvinyl)-cyclopropancarboxylat,

Difluor-3,4-dioxymethylen-benzyl-2,2-dimethyl-3-(2′,2′-dichlorvinyl)-cyclopropancarboxylat,

Difluor-2,3-dioxymethylen-benzyl-2,2-dimethyl-3-(2′,2′-dichlorvinyl)-cyclopropancarboxylat,

Difluor-3,4-dioxymethylen-6-chlorbenzyl-2,2-dimethyl-3-(2′,2′-dichlorvinyl)-cyclopropancarboxylat,

Difluor-3,4-dioxymethylen-6-brombenzyl-2,2-dimethyl-3-(2′,2′-dichlorvinyl)-cyclopropancarboxylat,

Difluor-3,4-dioxymethylen-2,5,6-trichlorbenzyl-2,2-dimethyl-3-(2',2'-dichlorvinyl)-cyclopropancarboxylat,
Difluor-3,4-dioxyäthylen-benzyl-2,2-dimethyl-3-(2',2'-dichlorvinyl)-cyclopropancarboxylat,
Trifluor-3,4-dioxyäthylen-benzyl-2,2-dimethyl-3-(2',2'-dichlorvinyl)-cyclopropancarboxylat,
Difluor-3,4-dioxyäthylen-6-chlorbenzyl-2,2-dimethyl-3-(2',2'-dichlorvinyl)-cyclopropancarboxylat,
Trifluor-3,4-dioxyäthylen-6-chlorbenzyl-2,2-dimethyl-3-(2',2'-dichlorvinyl)-cyclopropancarboxylat.
2-Difluormethoxybenzyl-4'-chlorphenyl-α-isopropylacetat,
3-Difluormethoxybenzyl-4'-chlorphenyl-α-isopropylacetat,
2-Tetrafluoräthoxybenzyl-4'-chlorphenyl-α-isopropylacetat,
3-Tetrafluoräthoxybenzyl-4'-chlorphenyl-α-isopropylacetat,
3-Hexafluorpropoxybenzyl-4'-chlorphenyl-α-isopropylacetat,
3-Difluormethoxy-4-chlorbenzyl-4'-chlorphenyl-α-isopropylacetat,
3,4-Bis-(difluormethoxy)-benzyl-4'-chlorphenyl-α-isopropylacetat,
3-Trifluormethylthio-benzyl-4'-chlorphenyl-α-isopropylacetat,
3-Trifluormethoxy-benzyl-4'-chlorphenyl-α-isopropylacetat,
3-Trifluormethylthio-benzyl-4'-chlorphenyl-α-isopropylacetat.
3-Difluormethoxy-cyano-benzyl-4'-chlorphenyl-α-isopropylacetat,
Difluor-3,4-dioxymethylen-benzyl-4'-chlorphenyl-α-isopropylacetat,
Difluor-2,3-dioxymethylen-benzyl-4'-chlorphenyl-α-isopropylacetat,
Difluor-3,4-dioxymethylen-6-chlorbenzyl-4'-chlorphenyl-α-isopropylacetat,
Difluor-3,4-dioxymethylen-6-brombenzyl-4'-chlorphenyl-α-isopropylacetat,
Difluor-3,4-dioxymethylen-2,5,6-trichlorbenzyl-4'-chlorphenyl-α-isopropylacetat,
Difluor-3,4-dioxyäthylen-benzyl-4'-chlorphenyl-α-isopropylacetat,
Trifluor-3,4-dioxyäthylen-benzyl-4'-chlorphenyl-α-isopropylacetat,
Difluor-3,4-dioxyäthylen-6-chlorbenzyl-4'-chlorphenyl-α-isopropylacetat,
Trifluor-3,4-dioxyäthylen-6-chlorbenzyl-4'-chlorphenyl-α-isopropylacetat.

Die Herstellung der erfindungsgemässen Benzylester der Formel I kann durch folgendes Reaktionsschema wiedergegeben werden:

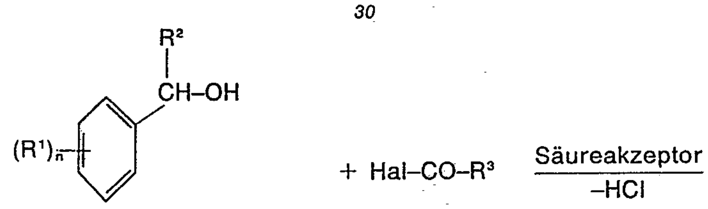


I

Verwendet man beispielsweise bei der Verfahrensvariante a) gemäss 2 (oben) 2,2-Dimethyl-3-(2',2'-dichlorvinyl)-cyclopropancarbonsäurechlorid und Difluor-3,4-methylendioxybenzylalkohol als Ausgangsmaterialien, so kann der Reaktionsverlauf durch das folgende Formelschema wiedergegeben werden:

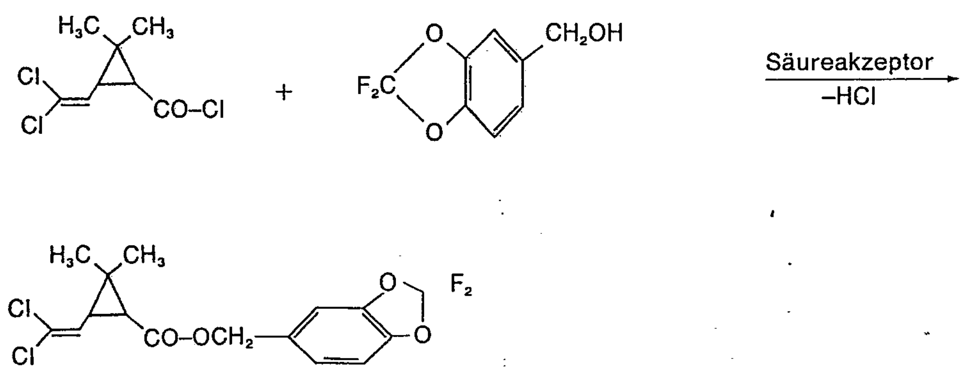


Die als Ausgangsprodukte zu verwendenden Carbonylhalogenide der Formel II sind bekannt und nach allgemein üblichen, in der Literatur beschriebenen Verfahren herstellbar (vgl. z.B. DT-A-2 365 555; 1 926 433 und 2 231 312).

Bevorzugt sind Verbindungen der Formel II in welcher $R^3$ die weiter oben angegebene bevorzugte oder besonders bevorzugte Bedeutung hat.

Als Beispiele für die als Ausgangsprodukte zu verwendenden Verbindungen der Formel II seien im einzelnen genannt:
2,2-Dimethyl-3-(2',2'-dichlorvinyl)-cyclopropancarbonsäurechlorid,
2,2-Dimethyl-3-(2',2'-dibromvinyl)-cyclopropancarbonsäurechlorid,
2,2-Dimethyl-3-(2',2'-dimethylvinyl)-cyclopropancarbonsäurechlorid,

α-Isopropyl-phenylessigsäurechlorid,
α-Isopropyl-4-fluorphenylessigsäurechlorid,
α-Isopropyl-4-chlorphenylessigsäurechlorid,
α-Isopropyl-4-bromphenylessigsäurechlorid,
α-Isopropyl-4-methylphenylessigsäurechlorid,
α-Isopropyl-4-äthylphenylessigsäurechlorid,
α-Isopropyl-4-n-propylphenylessigsäurechlorid,
α-Isopropyl-4-iso-propylphenylessigsäurechlorid,
α-Isopropyl-4-methoxyphenylessigsäurechlorid,
α-Isopropyl-4-äthoxyphenylessigsäurechlorid,
α-Isopropyl-4-methylthiophenylessigsäurechlorid,
α-Isopropyl-4-äthylthiophenylessigsäurechlorid,
α-Isopropyl-4-nitrophenylessigsäurechlorid,
α-Isopropyl-3-fluorphenylessigsäurechlorid,
α-Isopropyl-3-bromphenylessigsäurechlorid,
α-Isopropyl-3-chlorphenylessigsäurechlorid,
α-Isopropyl-3-methylphenylessigsäurechlorid,
α-Isopropyl-3-äthylphenylessigsäurechlorid,
α-Isopropyl-3-methoxyphenylessigsäurechlorid,
α-Isopropyl-3-äthoxyphenylessigsäurechlorid,
α-Isopropyl-3-methylthiophenylessigsäurechlorid,
α-Isopropyl-3-äthylthiophenylessigsäurechlorid,
α-Isopropyl-3,4-methylendioxyphenylessigsäurechlorid.

Die ebenfalls als Ausgangsprodukte zu verwendenden Alkohole der Formel III sind teilweise neu.

Die neuen Alkohole der Formel IIIa können nach den unter 4 (oben) angegebenen Verfahren hergestellt werden (Einzelheiten siehe weiter unten).

Bevorzugt werden Alkohole der Formel III verwendet in welcher $R^1$, $R^2$ und n die weiter oben angegebene bevorzugte oder besonders bevorzugte Bedeutung haben.

Als Beispiele für die als Ausgangsprodukte zu verwendenden Alkohole der Formel III seien im einzelnen genannt:

2-Difluormethoxy-benzylalkohol,
3-Difluormethoxy-benzylalkohol,
3-Trifluormethylthio-benzylalkohol,
3,4-Difluormethoxy-benzylalkohol,
3-Difluormethoxy-4-chlor-benzylalkohol,
3-Trifluormethoxy-benzylalkohol,
3-Trifluormethylthio-benzylalkohol,
Difluor-3,4-dioxymethylen-benzylalkohol,
2-Difluormethoxy-α-cyano-benzylalkohol,
3-Difluormethoxy-α-cyano-benzylalkohol,
2-Trifluormethylthio-α-cyano-benzylalkohol,
3,4-Difluormethoxy-α-cyano-benzylalkohol,
3-Difluormethoxy-4-chlor-α-cyano-benzylalkohol,
3-Trifluormethoxy-α-cyano-benzylalkohol,
3-Trifluormethylthio-α-cyano-benzylalkohol,
Difluor-3,4-dioxymethylen-α-cyano-benzylalkohol,
Difluor-3,4-dioxymethylen-α-äthinyl-benzylalkohol,
3-Difluormethoxy-α-äthinyl-benzylalkohol,
3-Difluormethylthio-benzylalkohol,
3-Difluormethylthio-α-cyano-benzylalkohol,
3-Tetrafluoräthoxy-benzylalkohol.

Zur Herstellung der erfindungsgemässen Verbindungen der Formel I gemäss 1 (oben) aus Alkoholen der Formel III und Carbonylhalogeniden der Formel II können als Säureakzeptoren alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines grösseren Bereichs variiert werden. Im allgemeinen arbeitet man zwischen 0 und 100 °C, vorzugsweise bei 15 bis 40 °C.

Die Umsetzung lässt man im allgemeinen bei Normaldruck ablaufen. Das Verfahren zur Herstellung der erfindungsgemässen Verbindungen wird bevorzugt unter Mitverwendung geeigneter Lösung- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol oder Äther, z.B. Diäthyl- und Dibutyläther, Dioxan, ferner Ketone, beispielsweise Aceton, Methyläthyl-, Methylisopropyl- und Methylisobutylketon, ausserdem Nitrile, wie Aceto- und Propionitril.

Zur Durchführung des Verfahrens setzt man die Ausgangskomponenten vorzugsweise in äquimolaren Verhältnissen ein. Ein Überschuss der einen oder anderen Komponente bringt keine wesentlichen Vorteile. Die Reaktionskomponenten werden im allgemeinen in einem der angegebenen Lösungsmittel zusammen gegeben und meist bei erhöhter Temperatur zur Vervollständigung der Reaktion eine oder mehrere Stunden gerührt. Anschliessend giesst man das Reaktionsgemisch in Wasser, trennt die organische Phase ab und wäscht diese mit Wasser nach. Nach dem Trocknen wird das Lösungsmittel im Vakuum abdestilliert.

Die Verbindungen fallen in Form von Ölen an, die sich zum Teil nicht unzersetzt destillieren lassen, jedoch durch sogenanntes «Andestillieren» d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex.

Ferner sind die erfindungsgemässen Verbindungen der Formel I durch Umsetzung der Carbonsäuresalze IV mit Benzylhalogeniden der Formel V erhältlich:

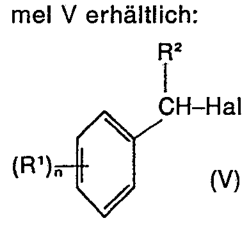

(I)

(V) + $MeO-CO-R^3$ →

(IV)

Verwendet man beispielsweise bei der Verfahrensvariante b) gemäss 2 (oben) das Kaliumsalz der 4-Chlorphenylessigsäure (das gegebenenfalls «in situ» aus Säure und KOH hergestellt werden kann) und 3-Difluormethoxy-benzylbromid als Ausgangsstoffe, so kann der Reaktionsverlauf durch das folgende Formelschema wiedergegeben werden:

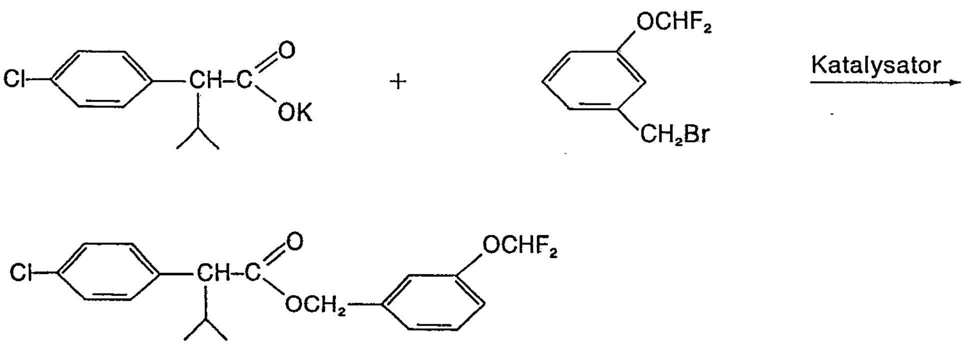


Die als Ausgangsprodukte zu verwendenden Carbonsäuren bzw. ihre Salze der Formel IV sind bekannt und liegen den Carbonylhalogeniden der Formel II zugrunde. Alle den Säurehalogeniden der Formel II zugrundeliegenden Säuren und Salze kommen somit für die hiergenannte Herstellungsweise in Betracht.

Bevorzugt werden Säuren und Salze der Formel IV verwendet in welcher der Rest $R^2$ die weiter oben angegebene bevorzugte oder besonders bevorzugte Bedeutung hat.

Im einzelnen seien die Säuren sowie deren Natrium, Kalium, Calcium oder Ammoniumsalze genannt, die den weiter oben aufgezählten Säurehalogeniden zugrundeliegen.

Die als Ausgangsprodukte verwendeten Benzylhalogenide der Formel V sind zum Teil bekannt. Die neuen Verbindungen können nach weiter unten angegebenen Verfahren erhalten werden. Bevorzugt werden Verbindungen der Formel V verwendet, in der $R^1$ und n die weiter oben angegebene bevorzugte oder besonders bevorzugte Bedeutung hat.

Folgende Benzylhalogenide seien im einzelnen genannt:

2-Difluormethoxybenzylchlorid,
3-Difluormethoxybenzylchlorid,
3-Difluormethoxy-4-chlorbenzylbromid,
3-Trifluormethoxy-benzylbromid,
Difluor-3,4-dioxymethylenbenzylchlorid,
Difluor-3,4-dioxymethylenbenzylbromid,
Difluor-3,4-dioxymethylen-6-chlorbenzylbromid,
Difluor-3,4-dioxymethylen-6-brombenzylbromid,
Difluor-3,4-dioxymethylen-α-cyanobenzylbromid,
Difluor-2,3-dioxymethylen-benzylchlorid,
Difluor-2,3-dioxymethylen-benzylbromid,
3-Tetrafluoräthoxy-benzylchlorid,
3-Tetrafluoräthoxy-benzylbromid,
Difluor-3,4-dioxymethylen-2,5,6-trichlorbenzylchlorid.

Zur Herstellung der erfindungsgemässen Derivate der Formel I aus den Carbonsäuresalzen der Formel IV und den Benzylhalogeniden der Formel V wird üblicherweise ein Lösungsmittel verwendet, wie beispielsweise Benzol, Toluol, Xylol, Chlorbenzol, Dichlorbenzol.

Die Salze der Carbonsäuren können direkt eingesetzt werden oder aber durch Zusatz von KOH oder NaOH in Form ihrer wässrigen Lösungen, oder in pulverisierter Form «in situ» hergestellt werden. Als Katalysatoren können quartäre Ammoniumsalze, wie beispielsweise Tetrabutylammoniumchlorid, Tetrabutylammoniumbromid, Benzyltriäthylammoniumchlorid oder Methyltrioctylammoniumchlorid verwendet werden.

Die Reaktionstemperatur kann innerhalb eines grösseren Bereichs variiert werden. Im allgemeinen arbeitet man zwischen 50 und 150 °C, vorzugsweise bei 80 bis 120 °C.

Zur Durchführung des Verfahrens setzt man die Ausgangskomponenten vorzugsweise in äquimolaren Verhältnissen ein. Ein Überschuss des Säuresalzes kann verwendet werden, um eine vollständige Umsetzung des Benzylhalogenids zu erreichen. Die überschüssige Säure kann aus der Wasserphase wiedergewonnen werden. Die Reaktion ist meist nach 1–5 Stunden beendet. Nach Abkühlen des Reaktionsgemisches wird mit Wasser versetzt, die organische Phase abgetrennt und neutral gewaschen. Anschliessend wird das Lösungsmittel im Vakuum abdestilliert und die Verbindungen der Formel I wie oben beschrieben gereinigt.

Wie bereits erwähnt sind die Alkohole der Formel IIIa neu. Sie lassen sich nach den unter 4 (oben) angegebenen Verfahren herstellen.

Bei Variante 4a wird für den Fall, dass in der gewünschten Verbindung der Formel IIIa $R^2$ für Wasserstoff steht der entsprechende Aldehyd mit Wasserstoff reduziert.

Als Reduktionsmittel kommen Wasserstoff in Anwesenheit von Katalysatoren oder Komplexe Metallhydride wie beispielsweise Natriumborhydrid oder Lithiumaluminiumhydrid in Frage. Die Durchführung der Reaktion erfolgt analog zu bekannten Verfahren (vgl. Organikum, VEB Deutscher Verlag der Wissenschaften, Berlin 1965, 5. Auflage, Seite 417; J. Am. Chem. Soc. 71, 122 (1949); 75, 199 (1953) und 76, 6116 (1954)).

Für den Fall, dass in der gewünschten Verbindung der Formel IIIa $R^2$ für CN steht wird der entsprechende Aldehyd mit HCN umgesetzt.

Die Durchführung der Reaktion erfolgt analog den bekannten Verfahren zur Herstellung von Cyanhydrinen (vgl. Organic Syntheses; Coll. Vol. I, 336; Houben-Weyl Band VIII, Seite 274f.).

Für den Fall, dass in der gewünschten Verbindung der Formel IIIa $R^2$ für $C_{1-4}$-Alkyl oder für Äthinyl steht, wird der entsprechende Aldehyd mit einer Grignard-Verbindung der Formel VII umgesetzt.

Die Durchführung der Reaktion erfolgt analog den in z.B. in Org. Synth. Coll. Vol. IV, Seite 792 beschriebenen Methoden.

Die Grignard-Verbindungen der Formel VII sind bekannt (vgl. z.B. die oben angegebene Literaturstelle).

Folgende Aldehyde der Formel VI seien im einzelnen genannt:
2-Difluormethoxy-benzaldehyd,
2-Trifluormethylthio-benzaldehyd,
3-Difluormethoxy-benzaldehyd,
3,4-Bis-difluormethoxy-benzaldehyd,
3-Difluormethoxy-4-chlor-benzaldehyd,
3-Trifluormethoxy-benzaldehyd,
3-Trifluormethylthio-benzaldehyd,
Difluor-3,4-dioxymethylen-benzaldehyd.

Die Aldehyde der Formel VI sind zum Teil bekannt (vgl. z.B. J. Org. Chem. 37 673 (1972), Z. obsc. Chim. 30, 3129 (1960)) oder können nach bekannten Verfahren hergestellt werden. Die Difluormethoxyverbindungen erhält man beispielsweise aus den entsprechenden Phenolen mit Difluorchlormethan in Gegenwart von Basen (vgl. z.B. DE-A-2 150 955, J. Org. Chem. 25, 2009 (1960)).

Die neuen Aldehyde der Formel VIa können erhalten werden, indem man die Verbindungen der Formel XIa gemäss 8 (oben) in an sich bekannter Weise in der Seitenkette zweifach halogeniert, bevorzugt chloriert, und die so erhaltenen Verbindungen in an sich bekannter Weise verseift.

Bei Variante 4b) werden Benzylamine der Formel VIIIa mit salpetriger Säure in Anwesenheit von Säuren, beispielsweise Essigsäure, umgesetzt.

Die dabei verwendeten Benzylamine der Formel VIIIa sind neu, man erhält sie durch Reduktion der entsprechenden Nitrile mit Wasserstoff analog zu bekannten Verfahren (Houben-Weyl, Band XI/1, Seite 577). Dabei werden die Nitrile z.B. durch Umsetzung der entsprechenden Bromverbindungen mit Kupfer(I)cyanid analog zu bekannten Verfahren erhalten (Houben-Weyl, Band VIII, S. 302).

Bei Variante 4c werden die Benzylhalogenide der Formel Va verseift.

Die Verseifung erfolgt dabei in an sich bekannter Weise mit wässrigen Basen, wie beispielsweise NaOH, KOH oder Alkalicarbonate, wie $Na_2CO_3$ oder $K_2CO_3$.

Die bei Verfahren 2b sowie 4c (oben) verwendeten Benzylhalogenide der Formel Va sind neu. Die neuen Verbindungen sind z.B. wie unter 6 (oben) angegeben erhältlich indem man nach im Prinzip bekannten Methoden Verbindungen der Formel IXa halogeniert, insbesondere bromiert oder chloriert.

Als Halogenierungsmittel kommen beispielsweise Chlor oder N-Chlor- oder N-Bromsuccinimid in Frage.

Die Chlorierung oder Bromierung der vorgenannten Verbindungen zu den entsprechenden Benzylchloriden oder -bromiden erfolgt in an sich bekannter Weise unter radikalischen Bedingungen mit Chlor, N-Chlorsuccinimid oder N-Bromsuccinimid in Lösungsmitteln wie beispielsweise Methylenchlorid, Chloroform, Tetrachlorkohlenstoff, Chlorbenzol, o-Difluorbenzol, vorzugsweise bei erhöhter Temperatur.

Wie bereits erwähnt, zeigen die Verbindungen der Formel I insektizide Wirksamkeit.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophaus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli,

Rhopalosiphum padi, Empoasca spp., Euscelis bilobatus, Nephotettix cincticeps, Lecanium corni, Saissetia oleae, Laodelphax striatelus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykoläther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel A
$LT_{100}$-Test für Dipteren
Testtiere: Musca domestica (resistent)
Zahl der Testtiere: 20
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z. B. die folgenden Verbindungen der Herstellungsbeispiele überlegene Wirkung gegenüber dem Stand der Technik: 3, 4, 6, 14, 8, 9, 1, 11, 12, 16.

Beispiel B
$LT_{100}$-Test für Dipteren
Testtiere: Aedes aegypti
Zahl der Testtiere: 20
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z. B. die folgenden Verbindungen der Herstellungsbeispiele überlegene Wirkung gegenüber dem Stand der Technik: 1, 3, 4, 6, 8, 9, 11, 12, 14, 16, 2.

Beispiel C
Mückenlarven-Test
Testtiere: Aedes aegypti, 4 Larven
Lösungsmittel: Aceton Gewichtsteile 99
Emulgator:
Benzylhydroxydiphenylpolyglykoläther Gewichtsteile 1

Zur Herstellung einer zweckmässigen Wirkstoffzubereitung löst man 2 Gewichtsteile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wässrigen Wirkstoffzubereitungen der gewünschten Konzentration in Gläser und setzt anschliessend etwa 25 Mückenlarven in jedes Glas ein.

Nach 24 Stunden wird der Abtötungsgrad in % bestimmt. Dabei bedeutet 100%, dass alle Larven abgetötet worden sind. 0% bedeutet, dass überhaupt keine Larven abgetötet worden sind.

Bei diesem Test zeigen z. B. die folgenden Verbindungen der Herstellungsbeispiele überlegene Wirkung gegenüber dem Stand der Technik: 3, 4, 6, 14, 8, 9, 1, 11, 12, 16.

Beispiel D
Laphygma-Test
Lösungsmittel:
3 Gewichtsteile Dimethylformamid
Emulgator:
1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z. B. die folgenden Verbindungen der Herstellungsbeispiele überlegene Wirksamkeit gegenüber dem Stand der Technik: 4, 8, 1, 9, 16, 12, 11, 3, 6, 14.

Herstellungsbeispiele

Beispiel 1

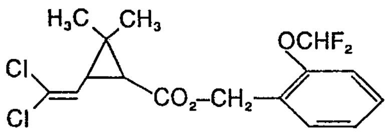

6,09 g (0,035 Mol) 2-Difluormethoxybenzylalkohol und 3,5 g (0,035 Mol) Triäthylamin werden in 50 ml Toluol gelöst und bei 20–25 °C zu einer Mischung von 7,95 g (0,35 Mol) 2,2-Dimethyl-3-(2′,2′-dichlor-vinyl)-cyclopropancarbonsäurechlorid in 100 ml Toluol getropft. Man rührt 3 h bei Raumtemperatur nach, giesst das Reaktionsgemisch in 150 ml Wasser, trennt die Toluolphase ab und wäscht sie mit 100 ml Wasser, trocknet mit Natriumsulfat und destilliert das Toluol i.V. ab. Letzte Lösungsmittelreste werden durch Andestillieren bei einer Badtemperatur von 60 °C/0,2 bis 1,0 Torr entfernt. Man erhält 10,9 g (81% d. Th.)

2-Difluormethoxybenzyl-2,2-dimethyl-3-(2′,2′-dichlorvinyl)-cyclopropancarboxylat

als viskoses Öl mit dem Brechungsindex $n_D^{20}$ = 1.5190.

Analog Beispiel 1 werden erhalten:

| Beispiel | Formel | Brechungsindex $n_D^{20}$ |
|---|---|---|
| 2 | 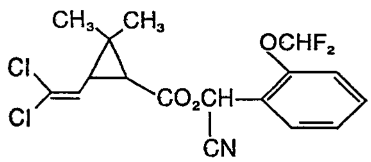 | 1.5191 |
| 3 | 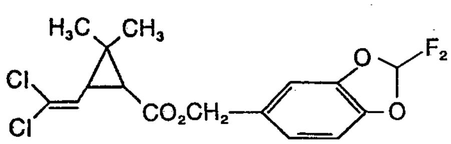 | 1.5070 |
| 4 | 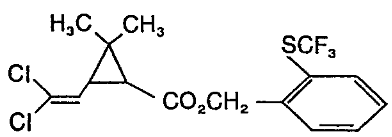 | 1.5260 |
| 5 | 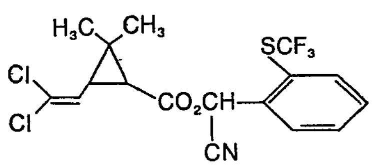 | 1.5240 |
| 6 | 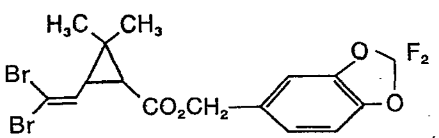 | 1.5230 |
| 7 | 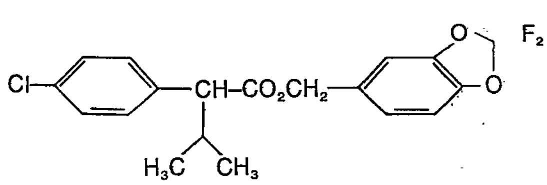 | 1.5170 |
| 8 | 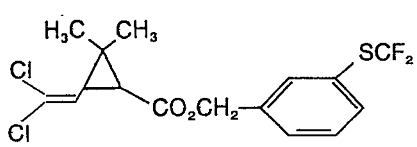 | 1.5154 |

| Beispiel | Formel | Brechungsindex $n_D^{20}$ |
|---|---|---|
| 9 | 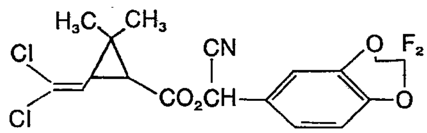 | 1.5062 |

Beispiel 10

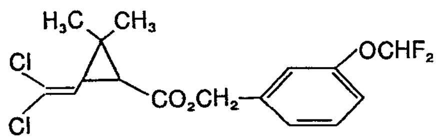

6,81 g (0,03 Mol) 3-Difluormethoxy-benzylbromid und 6,27 g (0,03 Mol) 2,2-Dimethyl-3-(2',2'-dichlorvinyl)-cyclopropancarbonsäure werden in 150 ml Toluol gelöst und anschliessend 0,5 g Tetrabutylammoniumbromid und 2 g pulverisierte KOH (technisch 87%ig) zugegeben und 2–3 h zum Sieden erhitzt. Nach dem Abkühlen giesst man das Reaktionsgemisch in 150 ml Wasser, trennt die Toluolphase ab und wäscht sie mit 100 ml Wasser; trocknet mit Natriumsulfat und destilliert das Voluol i.V. ab. Letzte Lösungsmittelreste werden durch Andestillieren bei einer Badtemperatur von 60–80 °C/0,2 bis 1 Torr entfernt. Man erhält 8,3 g (78% d. Th.)

3-Difluormethoxy-benzyl-2,2-dimethyl-3-(2',2'-dichlorvinyl)-cyclopropancarboxylat

als viskoses Öl mit dem Brechungsindex $n_D^{20}$ = 1.5110.

Beispiel 10a

Man arbeitet wie in Beispiel 9, verwendet jedoch 5,74 g (0,03 Mol) 3-Difluormethoxy-benzylchlorid statt 3-Difluormethoxybenzylbromid. Man erhält nach 4-stündigem Rückflusskochen 7,9 g (74% d. Th.)

3-Difluormethoxybenzyl-2,2-dimethyl-3-(2',2'-dichlorvinyl)-cyclopropancarboxylat.

Analog Beispiel 10 oder 10a werden erhalten:

| Beispiel | Formel | Brechungsindex $n_D^{20}$ |
|---|---|---|
| 11 | 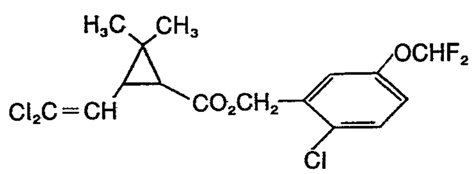 | 1.5200 |
| 12 | 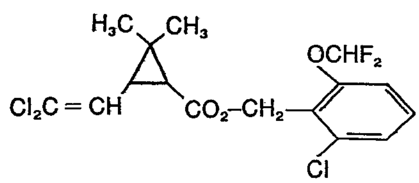 | 1.5210 |
| 13 | 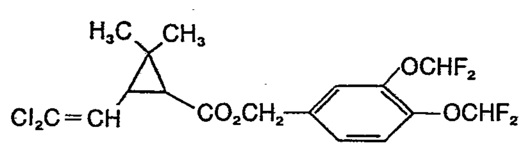 | 1.4960 |
| 14 | 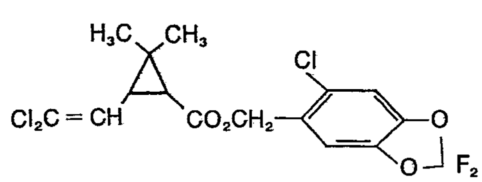 | 1.5110 |

| Beispiel | Formel | Brechungsindex $n_D^{20}$ |
|---|---|---|
| 15 | 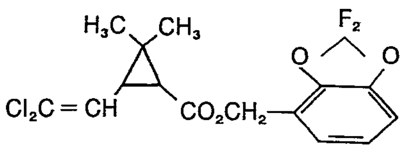 | 1.5080 |
| 16 | 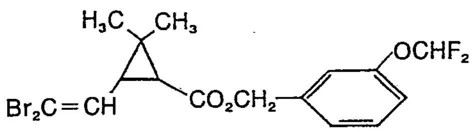 | 1.5280 |
| 17 | 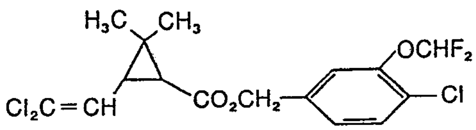 | 1.5154 |
| 18 | 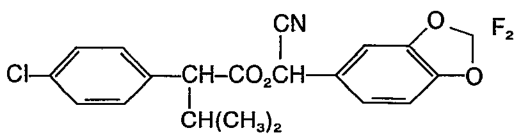 | 1.5150 |
| 19 | 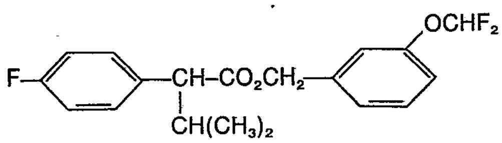 | 1.5136 |
| 20 | 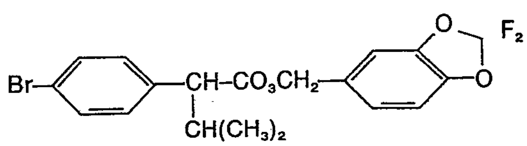 | 1.5205 |
| 21 | 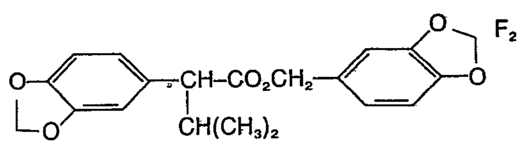 | 1.4992 |
| 22 | 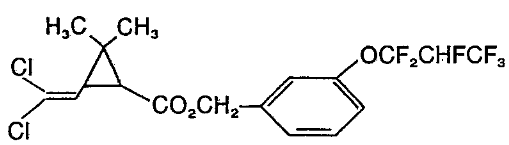 | 1.4780 |
| 23 | 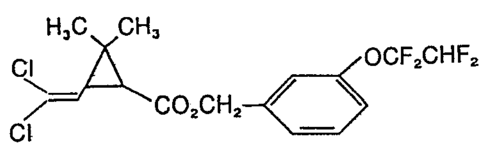 | 1.4950 |
| 24 | 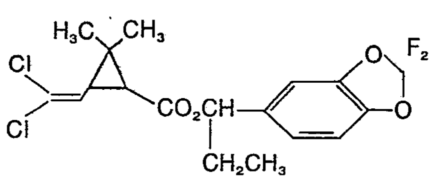 | 1.5164 |

| Beispiel | Formel | Brechungsindex $n_D^{20}$ |
|---|---|---|
| 25 | 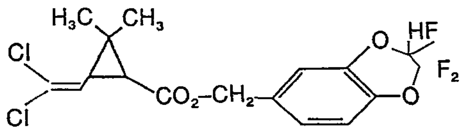 | 1.5090 |
| 26 | 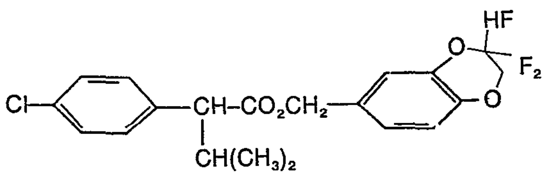 | 1.5149 |
| 27 | 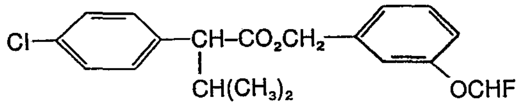 | 1.5193 |
| 28 | 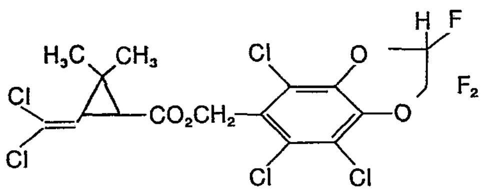 | 1.543 |
| 29 | 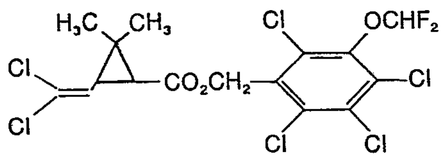 | 1.546 |
| 30 | 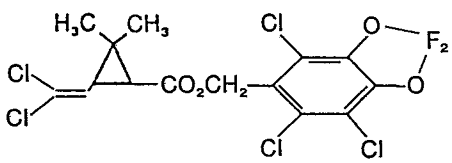 | 1.538 |

Die folgenden Verbindungen werden analog Beispiel 1 erhalten:

| | | |
|---|---|---|
| 31 | 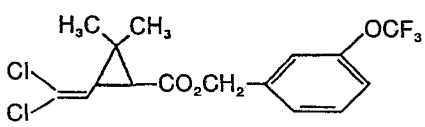 | 1.493 |
| 32 | 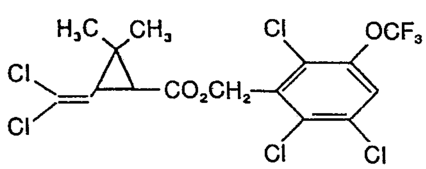 | 1.532 |
| 33 | 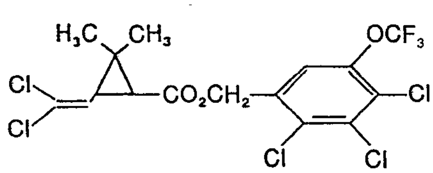 | 1.530 |

| Beispiel | Formel | Brechungsindex $n_D^{20}$ |
|---|---|---|
| 34 | 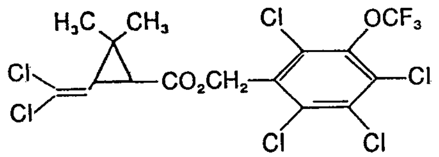 | 1.538 |
| 35 | 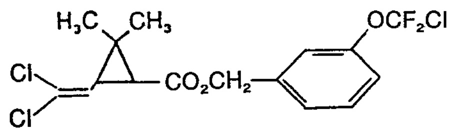 | 1.512 |
| 36 | 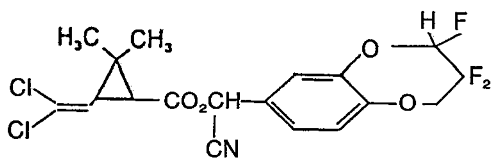 | 1.517 |

Herstellung der Ausgangsstoffe

1. 2-Difluormethoxybenzylalkohol

Man löst 51,5 g (0,3 Mol) 2-Difluormethoxybenzaldehyd in 100 ml Äthanol und tropft 6,25 g Natriumborhydrid in 100 ml Äthanol bei 25–35 °C zu. Anschliessend rührt man nach bis Raumtemperatur erreicht ist, verdünnt mit Wasser und säuert mit 25%iger $H_2SO_4$ an. Nach zweimaligem Extrahieren mit Methylenchlorid wird die organische Phase getrocknet und das Lösungsmittel abdestilliert. Man erhält ein farbloses Öl in nahezu quantitativer Ausbeute, das nach einiger Zeit erstarrt. Der 2-Difluormethoxybenzylalkohol schmilzt bei 42 °C.

2. 2- und 3-Trifluormethylthiobenzylalkohol

werden analog dem vorstehenden Beispiel aus 2-Trifluormethylthiobenzaldehyd erhalten.

3. 2-Difluormethoxy-α-cyano-benzylalkohol

7,2 g Kaliumcyanid werden unter Kühlung in 30 ml Wasser und 6 ml Äthanol gelöst. Dann wird unter Kühlung bei 0–5 °C 16 g 2-Difluormethoxybenzaldehyd zugegeben. Nach 20 Min. Nachrühren wird zwischen 0 und 10 °C eine Mischung aus 7 ml konz. $H_2SO_4$ und 18 ml Wasser zugetropft. Man rührt noch 2 h nach und lässt dabei auf Raumtemperatur kommen. Nach zweimaligem Extrahieren mit Methylenchlorid wird die organische Phase getrocknet und das Lösungsmittel abdestilliert. Man erhält 2-Difluormethoxy-α-cyano-benzalkohol als farbloses Öl, dessen Struktur durch ein Kernresonanzspektrum bestätigt wird. Die Ausbeute ist praktisch quantitativ.

4. Analog vorstehendem Beispiel erhält man 2-Trifluormethylthio-α-cyano-benzylalkohol aus 2-Trifluormethylthio-benzaldehyd und Difluor-3,4-dioxymethylen-α-cyano-benzylalkohol aus Difluor-3,4-dioxymethylen-benzaldehyd.

5. Analog Beispiel 1, erhält man aus Difluor-3,4-dioxymethylenbenzaldehyd durch Reduktion mit Natriumborhydrid Difluor-3,4-dioxymethylenbenzylalkohol. Das NMR-Spektrum (in $CDCl_3$) bestätigt die Struktur: 3,7 ppm (s, OH); 4,6 ppm (s, $CH_2$); 7,0 ppm und 7,05 ppm (3. aromat. H).

6. Difluor-3,4-methylendioxy-toluol

Im Reaktionsgefäss werden bei 0 °C 200 ml HF (wasserfrei) vorgelegt und 190 g Dichlor-3,4-methylendioxy-toluol (vgl. J. Chem. Soc. 93, 563) zugetropft. Nach Ende der Chlorwasserstoffentwicklung wird auf 20 °C erwärmt, 1 h nachgeführt und anschliessend die überschüssige Flusssäure bei vermindertem Druck abdestilliert. Difluor-3,4-methylendioxy-toluol siedet bei 74–78 °C/52 mm ($n_D^{20}$ = 1.492).

7. 1-n-Propyl-difluor-3,4-methylendioxy-benzol erhält man analog der vorstehenden Vorschrift. Kp = 80 °C/15 mm ($n_D^{20}$ = 1.4540).

8. Difluor-3,4-methylendioxy-benzylbromid

172 g (1 Mol) Difluor-3,4-methylendioxytoluol, 180 g N-Bromsuccinimid und eine Spatelspitze Azobisisobutyronitril werden mit 1000 ml $CCl_4$ gemischt und 5 h zum Sieden erhitzt. Nach dem Abkühlen wird abfiltriert, mit etwas $CCl_4$ nachgewaschen und das Filtrat destilliert. Man erhält 180 g (72% d. Th.) Difluor-3,4-methylendioxy-benzylbromid vom Kp = 180–111 °C/15 mm. Brechungsindex $n_D^{20}$ = 1.518.

9. Analog vorstehendem Beispiel erhält man 1-Brom-1-(difluor-3,4-methylendioxy)-phenylpropan vom Kp = 70–73 °C/0,3 mm.

10. Difluor-3,4-methylendioxy-6-chlor-benzyl bromid

In eine Lösung von 34,4 g (0,2 Mol) Difluor-3,4-methylendioxy-toluol in 40 ml Methylenchlorid

werden bei $-10\,°C$ bis $-5\,°C$ 14 g Chlor eingeleitet. Man lässt auf Raumtemperatur kommen und fraktioniert im Wasserstrahlvakuum. Man erhält 31 g Difluor-3,4-methylen-dioxy-6-chlor-toluol vom Kp = 80–84 °C/15 mm, die in 150 ml Tetrachlorkohlenstoff gelöst werden. Nach Zugabe von 33 g N-Brom-succinimid und einer Spatelspitze Azibisisobutyronitril erhitzt man 5 h zum Sieden. Man erhält 30 g Difluor-3,4-methylendioxy-6-chlorbenzylbromid vom Kp = 74–75 °C/0,25 mm Brechungsindex $n_D^{20}$ = 1.5334.

11. Trifluor-3,4-dioxyäthylen-benzylbromid

Man löst 124 g 4-Methylbenzkatechin in 300 ml Tetramethylensulfon und fügt 110 g KOH hinzu. Anschliessend leitet man bei 100° bis 110 °C 170 g Trifluorchloräthylen ein. Nach dem Abkühlen destilliert man über eine Kolonne im Wasserstrahlvakuum. Man erhält 132 g Trifluor-3,4-dioxyäthylen-toluol vom Kp 70–72 °C/12 mm $n_D^{20}$ = 1.4565.

50 g werden mit 50 g N-Bromsuccinimid in 150 ml $CCl_4$ analog vorstehender Vorschrift bromiert (8 h Rückfluss). Das Trifluor-3,4-dioxyäthylen-benzylbromid siedet bei 123–124 °C/13 mm; Brechungsindex $n_D^{20}$ = 1.5165.

12. 4-Methyl-2,2-difluorbenzodioxol

Im Reaktionsgefäss werden bei 0 °C 200 ml HF vorgelegt und 100 g 4-Methyl-2,2-dichlorbenzodioxol zugetropft. Nach Ende der Chlorwasserstoffentwicklung wird noch auf 20 °C erwärmt, 1 Stunde nachgerührt und anschliessend die überschüssige Flusssäure bei vermindertem Druck abdestilliert. Das 4-Methyl-2,2-difluorbenzodioxol hat einen Siedepunkt von 42–45 °C bei 11 mm ($n_D^{20}$: 1,4515).

Das 4-Brommethyl-2,2-difluorbenzodioxol siedet bei 93–96 °C/10 mm ($n_D^{20}$ = 1,5115).

13. 5-Propyl-2,2-difluorbenzodioxol

Bei $-2\,°C$ werden 100 ml wasserfreie Flusssäure im Reaktionsgefäss vorgelegt und anschliessend 110 g 5-Propyl-2,2-dichlorbenzodioxol zugetropft. Nach analoger Durchführung wie im Beispiel 15a erhält man 67 g 5-Propyl-2,2-difluorbenzodioxol mit einem Siedepunkt von 80–83 °C bei 15 mm ($n_D^{20}$: 1.4540).

14. Difluormethylen-3,4-dioxybenzoesäure

100 ml wasserfreie Flusssäure werden bei $-3\,°C$ vorgelegt und dann 55 g 5-Chlorcarbonyl-2,2-dichlorbenzodioxol zugetropft. Nach Ende der Zugabe wird noch auf 20 °C erwärmt und bis zum Ende der Chlorwasserstoffentwicklung gerührt. Dann wird die überschüssige Flusssäure abdestilliert und der Rückstand in 200 ml 5%ige Natronlauge eingerührt. Die Lösung wird filtriert und anschliessend mit Salzsäure angesäuert. Die ausgefallene Difluormethylen-3,4-dioxybenzoesäure wird abgesaugt und getrocknet. Man erhält 35 g Säure mit einem Schmelzpunkt von 153–154 °C.

15. 2,2-Difluorbenzodioxol

a) In einem Reaktionsgefäss aus $V_4A$ mit Rührer, Rückflusskühler und Tropftrichter werden bei $-10\,°C$ 600 g wasserfreie Flusssäure vorgelegt. Dann werden 612 g 2,2-Dichlorbenzodioxol unter Feuchtigkeitsausschluss in ca. 2 h zugetropft. Es setzt sofort Chlorwasserstoffentwicklung ein. Das Gas wird durch eine Ableitung vom Rückflusskühler in eine Vorlage mit Wasser geleitet und absorbiert. Nach Ende der Zugabe wird die Temperatur auf 18°–20 °C erhöht und noch 1 Stunde bis Ende der Gasentwicklung nachgerührt. Die überschüssige Flusssäure wird nun über eine Kolonne abdestilliert und in einer gekühlten Vorlage aufgefangen. Anschliessend destilliert man bei 100 mm Druck mit einem Siedepunkt von 65–70 °C 394 g 2,2-Difluorbenzodioxol über ($n_D^{20}$: 1.4430). Die Ausbeute beträgt 78% d. Th.

b) Zu 200 ml wasserfreier Flusssäure im Reaktionsgefäss aus $V_4A$ tropft man unter Rühren eine Lösung von 150 g 2,2-Dichlorbenzodioxol in 200 ml Methylenchlorid. Die Reaktion setzt bei 0 °C sofort ein. Man lässt bei 0 °C ausreagieren, erhöht dann die Temperatur auf 20 °C und rührt für eine weitere Stunde. Dann destilliert man die überschüssige Flusssäure und das Lösungsmittel bei vermindertem Druck ab und destilliert anschliessend das 2,2-Difluorbenzodioxol über. Man erhält 85 g Produkt, das entspricht 68% d. Th.

16. 2,2-Difluor-5-chlor-benzodioxol wird analog Beispiel 15a erhalten. Kp = 57 °C/14 mm ($n_D^{20}$ = 1,4712).

17. 2,2-Difluor-1,4-benzodioxen

110 g Brenzkatechin werden zusammen mit 70 g Kaliumhydroxid in 300 ml Tetramethylensulfon vorgelegt und unter Rühren innerhalb von 30 Min. auf 100 °C erhitzt. Bei einer Temperatur von 100–110 °C werden 140 g 1,1-Difluor-2-chloräthylen eingeleitet (Dauer ca. 3 Stunden). Anschliessend wird bei 15 mm das Produkt über eine kleine Kolonne in eine gute gekühlte Vorlage destilliert. Man erhitzt hierbei bis zu einer Innentemperatur von 100 °C. Der Inhalt der Vorlage wird in einem Scheidetrichter überführt und die organische Phase von der wässrigen abgetrennt. Man erhält 112 g ≙ 65% d. Th. an 2,2-Difluor-1,4-benzodioxen mit einem Brechungsindex $n_D^{20}$: 1.4802, das nach GC-Analyse rein ist.

18. 6-Methyl-2,2,3-trifluor-1,1-benzodioxen

124 g 4-Methylbrenzkatechin werden zusammen mit 110 g Kaliumhydroxid in 300 ml Tetramethylensulfon bei 110 °C vorgelegt. Es werden dann innerhalb 4 Stunden 170 g Trifluorchloräthylen eingeleitet. Der Ansatz wird anschliessend bei 15 mm über eine Kolonne destilliert, wobei bis zu einer Übergangstemperatur von 85 °C abgenommen wurde. Nach Abtrennen der wässrigen Phase in der Vorlage wird das Produkt erneut destilliert. Man erhält bei einem Siedepunkt $Kp_{12}$: 70–2 °C ($n_D^{20}$: 1.4565) 133 g ≙ 65% d. Th. 6-Methyl-2,2,3-trifluor-1,4-benzodioxen.

19. 2,2,3-Trifluor-1,4-benzodioxen

In 600 ml Tetramethylensulfon werden 220 g Brenzkatechin und 130 g Natriumhydroxid bei

95–105 °C vorgelegt und bei dieser Temperatur unter Rühren 330 g Trifluorchloräthylen eingeleitet. Man destilliert anschliessend den Ansatz bei 15 mm über eine Kolonne und fängt eine Fraktion von $Kp_{15}$: 20 bis 60 °C in einer gut gekühlten Vorlage auf. Nachdem die $H_2O$-Phase abgetrennt wurde, verbleiben 332 g reines 2,2,3-Trifluor-1,4-benzodioxen vom Siedepunkt $Kp_{12}$: 54–5 °C, $n_D^{20}$: 1.4525 mit einer Ausbeute von 87% d. Th.

20. 2,2,3-Trifluor-5,7,8-trichlor-6-chlormethyl-1,4-benzodioxen

60 g 6-Methyl-2,2,3-trifluor-benzodioxen werden mit 1 g $FeCl_3$ bei 25 °C vorgelegt und Chlor eingeleitet. Man lässt die Temperatur langsam bis auf 80 °C ansteigen und chloriert bis zur Sättigung weiter. Nach einem kleinen Vorlauf destillieren bei 120–125 °C/0,15 mm 71 g Produkt vom Schmelzpunkt 84–86 °C.

21. 3-Difluormethoxy-2,4,5,6-tetrachlorbenzylchlorid

100 g Difluormethoxy-toluol werden mit 2 g Jod bei 25–30 °C chloriert. Nach Einleiten von ca. 150 g Chlor verfestigt sich die Reaktionsmischung und man fügt 50 ml $CH_2Cl_2$ hinzu und chloriert bis zur Sättigung bei 40 °C weiter. Nach Abdestillieren des Lösungsmittels verbleibt ein Festprodukt, das auf einen Filter aufgebracht und anschliessend mit Cyclohexan gewaschen wird. Es fallen 140 g 3-Difluormethoxy-tetrachlor-toluol an (Schmelzpunkt 86–88 °C). Diese werden in 250 ml o-Dichlorbenzol gelöst und bei 175–185 °C unter UV-Bestrahlung mit ca. 150 g Chlor chloriert. Nach Destillation (Kp = 140–142 °C/1,2 mm) werden 112 g 3-Difluormethoxy-2,4,5,6-tetrachlorbenzylchlorid erhalten.

22. 4,6,7-Trichlor-2,2-difluor-5-chlormethylbenzyldioxol

162 g 5-Methyl-2,2-difluor-benzodioxol werden mit 1 g FeS bei 10 °C vorgelegt und Chlor eingeleitet. Wenn die Chloraufnahme abnimmt, wird die Temperatur langsam gesteigert, bis bei 70 °C Sättigung erreicht ist. Nach einem Vorlauf gehen bei der Destillation 210 g 5-Chlormethyltrichlor-2,2-difluor-benzodioxol vom Kp = 104–105 °C/0,3 mm über. Schmelzpunkt 48–50 °C.

23. 3-Trifluormethoxy-benzylalkohol

wird durch Reduktion von 3-Trifluormethoxybenzoylfluorid mit $NaBH_4$ in Dioxan erhalten. Kp = 95–97 °C/15 mm; $n_D^{20}$ = 1,4485. Analog erhält man aus 3-Chlordifluormethoxybenzoylfluorid 3-Chlordifluormethoxy-benzylalkohol: Kp = 123 °C/14 mm; $n_D^{20}$ = 1,4840.

24. 2,2,3-Trifluor-6-hydroxy-cyano-methyl-1,4-benzodioxen

Man erhitzt 40 g Hexamethylentetramin in 50 ml Wasser auf 100 °C und tropft 40 g 6-Chlormethyl-2,2,3-trifluorbenzodioxen zu. Nach 1 Stunde bei 100 °C werden noch 100 ml Wasser und 100 ml konzentrierter Salzsäure zugegeben und für 2 Stunden bei 100 °C gerührt. Durch anschliessende Wasserdampfdestillation erhält man 20 g 6-Formyl-2,2,3-trifluor-1,4-benzodioxen vom Kp 119–120 °C/15 mm; $n_D^{20}$ = 1,5001), das analog Beispiel 3 auf Seite 61 mit KCN in das Cyanhydrin überführt wird.

25. 3-Trifluormethoxy-tetra (und tri-)chlorbenzylalkohol

200 g 3-Trifluormethoxy-benzoylfluorid werden mit 5 g FeS bei Rückfluss (167 °C) vorgelegt und Chlor eingeleitet. Die Chloraufnahme ist anfangs schwach, nimmt aber in dem Masse zu, wie die Temperatur steigt. Bei 180 °C wird bis zur Sättigung chloriert.

Diese Rohmischung wird in 500 ml Dioxan bei 20 °C mit 65 g Natriumborhydrid reduziert und nach Hydrolyse mit $CH_2Cl_2$ extrahiert und destilliert.

Bei $Kp_{15}$: 148–152 °C geht eine Fraktion über, die nach Stehen bei 20 °C zum Teil kristallisiert. Die Kristalle werden abgesaugt und mit Hexan gewaschen. F: 50–2 °C, nach $H^1$-NMR-Spektrum liegt 5-Trifluormethoxy-2,3,4-trichlor-benzylalkohol vor.

Nach Abdestillieren des Hexans verbleibt ein Öl, das nach $H^1$-NMR zu 80% aus 3-Trifluormethoxy-2,5,6-trichlorbenzylalkohol besteht.

Die zweite Fraktion bei der Destillation ($Kp_{13}$: 170–5 °C, F: 76–8 °C) besteht aus 3-Trifluormethoxy-2,4,5,6-tetrachlorbenzylalkohol.

## Patentansprüche

1. Benzylester der Formel I

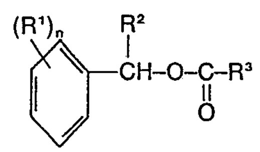

(I)

in welcher

n für 1–5 steht und

$R^1$ für gleiche oder verschiedene Reste aus der Gruppe Wasserstoff, $C_{1-6}$-Alkyl, $C_{1-6}$-Halogenalkyl mit 3–6 Halogenatomen, $C_{1-6}$-Alkoxy, $C_{1-6}$-Halogenalkoxy mit 1–6 Halogenatomen, $C_{1-6}$-Alkylmercapto, $C_{1-6}$-Halogenalkylmercapto mit 1–6 Halogenatomen, Halogen, gegebenenfalls substituiertes Phenyl, gegebenenfalls substituiertes Phenoxy, steht oder zwei benachbarte Reste gemeinsam mit den angrenzenden C-Atomen einen gegebenenfalls substituierten akondensierten Benzolring oder ein oder mehrfach durch Fluor substituierte Sauerstoff enthaltende heterocyclische Fünf- oder Sechsringe bilden. Dabei muss in Formel I entweder einer der Reste $R^1$ für Fluoralkoxy oder Fluoralkylmercapto stehen oder zwei benachbarte Reste müssen gemeinsam mit den angrenzenden C-Atomen ein oder mehrfach durch Fluor substituierte Sauerstoff enthaltende heterocyclische Fünf- oder Sechsringe bilden,

$R^2$ für Wasserstoff, $C_{1-4}$-Alkyl, Cyan oder Ethinyl steht,

$R^3$ für Reste der Formel

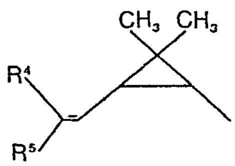

oder

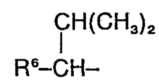

$R^4$ und $R^5$ für Chlor oder Brom stehen,

$R^6$ für gegebenenfalls durch Halogen, Alkyl, Alkylthio, Alkoxy mit jeweils 1–4 C-Atomen, Nitro, Methylendioxy substituiertes Phenyl steht.

2. Verfahren zur Herstellung der Benzylester der Formel I

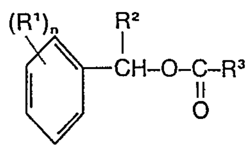

(I)

in welcher

n für 1–5 steht und

$R^1$ für gleiche oder verschiedene Reste aus der Gruppe Wasserstoff, $C_{1-6}$-Alkyl, $C_{1-6}$-Halogenalkyl mit 3–6 Halogenatomen, $C_{1-6}$-Alkoxy, $C_{1-6}$-Halogenalkoxy mit 1–6 Halogenatomen, $C_{1-6}$-Alkylmercapto, $C_{1-6}$-Halogenalkylmercapto mit 1–6 Halogenatomen, Halogen, gegebenenfalls substituiertes Phenyl, gegebenenfalls substituiertes Phenoxy, steht oder zwei benachbarte Reste gemeinsam mit den angrenzenden C-Atomen einen gegebenenfalls substituierten ankondensierten Benzolring oder ein oder mehrfach durch Fluor substituierte Sauerstoff enthaltende heterocyclische Fünf- oder Sechsringe bilden. Dabei muss in Formel I entweder einer der Reste $R^1$ für Fluoralkoxy oder Fluoralkylmercapto stehen oder zwei benachbarte Reste müssen gemeinsam mit den angrenzenden C-Atomen ein oder mehrfach durch Fluor substituierte Sauerstoff enthaltende heterocyclische Fünf- oder Sechsringe bilden,

$R^2$ für Wasserstoff, $C_{1-4}$-Alkyl, Cyan oder Ethinyl steht,

$R^3$ für Reste der Formel

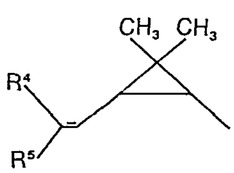

oder

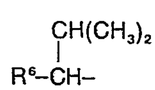

$R^4$ und $R^5$ für Chlor oder Brom stehen,

$R^6$ für gegebenenfalls durch Halogen, Alkyl, Alkylthio, Alkoxy mit jeweils 1–4 C-Atomen, Nitro, Methylendioxy substituiertes Phenyl steht,

dadurch gekennzeichnet, dass man

a) Carbonylhalogenide der Formel II

$$\text{Hal–CO–R}^3 \quad \text{(II)}$$

in welcher

$R^3$ die (oben) angegebene Bedeutung hat, und

Hal für Halogen, vorzugsweise Chlor, steht,

mit Benzylalkoholen der Formel III

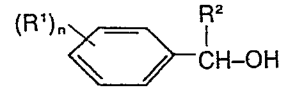

(III)

in welcher

$R^1$, n und $R^2$ die oben angegebene Bedeutung haben,

gegebenenfalls in Gegenwart eines Säureakzeptors und gegebenenfalls in Gegenwart eines Lösungsmittels umsetzt, oder

b) Carbonsäuresalze der Formel IV

$$\text{MeO–CO–R}^3 \quad \text{(IV)}$$

in welcher

Me K oder Na bedeutet und

$R^3$ die oben angegebene Bedeutung hat,

mit Benzylhalogeniden der Formel V

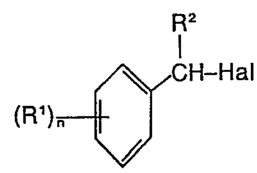

(V)

in welcher

$R^1$, n und $R^2$ die oben angegebene Bedeutung haben und

Hal Chlor oder Brom bedeutet,

gegebenenfalls in Gegenwart eines Lösungsmittels und gegebenenfalls in Gegenwart eines quartären Ammoniumsalzes umsetzt.

3. Benzylalkohole der Formel IIIa

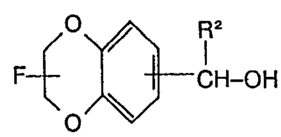

(IIIa)

in welcher

$R^2$ die in Anspruch 1 angegebene Bedeutung hat und wobei die übrigen Stellen des Benzolrings durch Halogen substituiert sein können.

4. Verfahren zur Herstellung der Benzylalkohole der Formel IIIa gemäss Anspruch 3, dadurch gekennzeichnet, dass man

a) Aldehyde der Formel VIa

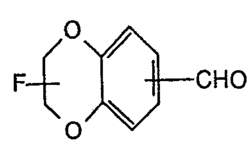

(VIa)

in welcher

die übrigen Stellen des Benzolrings durch Halogen substituiert sein können.

für den Fall, dass $R^2$ im Benzylalkohol für Wasserstoff steht reduziert, oder für den Fall, dass $R^2$ im Benzylalkohol für CN steht mit HCN umsetzt,

oder für den Fall, dass $R^2$ im Benzylalkohol für $C_{1-4}$-Alkyl oder Ethinyl steht mit einer Grignard-Verbindung der Formel VII

$$\text{R}^2\text{-Mg-Hal} \quad \text{(VII)}$$

in welcher

$R^2$ für $C_{1-4}$-Alkyl oder Ethinyl steht umsetzt, oder

b) dass man Benzylamine der Formel VIIIa

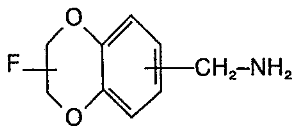

(VIIIa)

in welcher

die übrigen Stellen des Benzolrings durch Halogen substituiert sein können,

mit Natrium- oder Kaliumnitrit in Gegenwart einer Säure umsetzt oder

c) dass man ein Benzylhalogenid der Formel Va

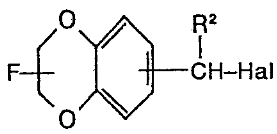

(Va)

in welcher

$R^2$ die in Anspruch 1 angegebene Bedeutung besitzt, Hal für Halogen steht und die übrigen Stellen des Benzolrings durch Halogen substituiert sein können, mit wässrigen Basen verseift.

5. Benzylhalogenide der Formel Va

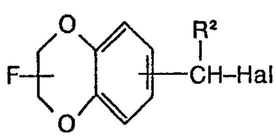

(Va)

in welcher

$R^2$ die in Anspruch 1 angegebene Bedeutung besitzt Hal für Halogen steht und die übrigen Stellen des Benzolrings durch Halogen substituiert sein können.

6. Verfahren zur Herstellung der Benzylhalogenide der Formel Va gemäss Anspruch 5, dadurch gekennzeichnet, dass man Verbindungen der Formel IXa

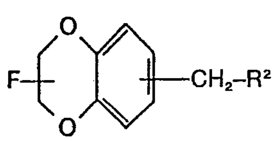

(IXa)

in welcher

$R^2$ die in Anspruch 1 angegebene Bedeutung hat und die übrigen Stellen des Benzolrings durch Halogen substituiert sein können,

in der Seitenkette auf an sich bekannte Weise halogeniert.

7. Aldehyde der Formel VIa

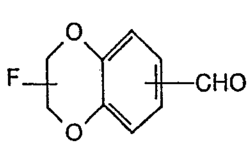

(VIa)

in welcher

die übrigen Stellen des Benzolrings durch Halogen substituiert sein können.

8. Verfahren zur Herstellung der Aldehyde der Formel VIa gemäss Anspruch 7, dadurch gekennzeichnet, dass man in Verbindungen der Formel XIa

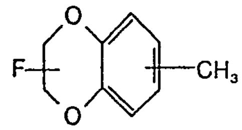

(XIa)

in welcher

die übrigen Stellen des Benzolrings durch Halogen substituiert sein können,

die $CH_3$-Gruppe in an sich bekannter Weise zur $-CH\ Hal_2$-Gruppe halogeniert und anschliessend in üblicher Weise zum Aldehyd der Formel VI verseift.

9. Benzylamine der Formel VIIIa

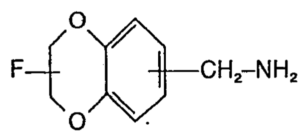

(VIIIa)

in welcher

die übrigen Stellen des Benzolrings durch Halogen substituiert sein können.

10. Verfahren zur Herstellung der Benzylamine der Formel VIIIa gemäss Anspruch 9, dadurch gekennzeichnet, dass man eine Verbindung der Formel XIIa

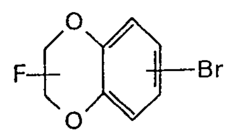

(XIIa)

in welcher

die übrigen Stellen des Benzolrings durch Halogen substituiert sein können,

in einer ersten Stufe mit einem Cyanidsalz umsetzt und das dabei erhaltene Nitril der Formel XIIIa

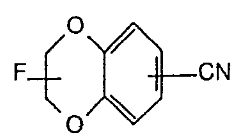

(XIIIa)

in welcher

die übrigen Stellen des Benzolrings durch Halogen substituiert sein können,

in an sich bekannter Weise hydriert.

11. Insektizide, gekennzeichnet durch einen Gehalt an mindestens einem Benzylester der Formel I gemäss Anspruch 1.

12. Verwendung von Benzylestern der Formel I gemäss Anspruch 1 zur Bekämpfung von Insekten.

**Claims**

1. Benzyl esters of the formula I

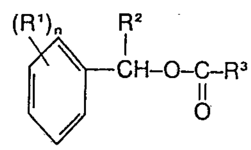

(I)

in which

n represents 1–5 and

$R^1$ represents identical or different radicals from the group comprising hydrogen, $C_{1-6}$-alkyl, $C_{1-6}$-halogenoalkyl with 3–6 halogen atoms, $C_{1-6}$-alkoxy, $C_{1-6}$-halogenoalkoxy with 1–6 halogen atoms, $C_{1-6}$-alkylmercapto, $C_{1-6}$-halogenoalkylmercapto with 1–6 halogen atoms, halogen, optionally substituted phenyl or optionally substituted phenoxy, or two adjacent radicals, together with the adjacent C atoms, form an optionally substituted fused-on benzene ring or heterocyclic five- or six-membered rings which contain oxygen and are mono- or polysubstituted by fluorine. In formula I either one of the radicals $R^1$ must represent fluoroalkoxy or fluoroalkylmercapto or two adjacent radicals must form, together with the adjacent C atoms, hererocyclic five- or six-membered rings which contain oxygen and are mono- or polysubstituted by fluorine,

$R^2$ represents hydrogen, $C_{1-4}$-alkyl, cyano or ethynyl,

$R^3$ represents radicals of the formula

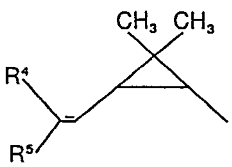

or

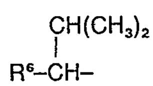

$R^4$ and $R^5$ represent chlorine or bromine, and

$R^6$ represents phenyl which is optionally substituted by halogen, alkyl, alkylthio or alkoxy, each with 1–4 C atoms, nitro or methylenedioxy.

2. Process for the preparation of the benzyl esters of the formula I

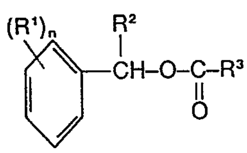

(I)

in which

n represents 1–5 and

$R^1$ represents identical or different radicals from the group comprising hydrogen, $C_{1-6}$-alkyl, $C_{1-6}$-halogenoalkyl with 3–6 halogen atoms, $C_{1-6}$-alkoxy, $C_{1-6}$-halogenoalkoxy with 1–6 halogen atoms, $C_{1-6}$-alkylmercapto, $C_{1-6}$-halogenoalkylmercapto with 1–6 halogen atoms, halogen, optionally substituted phenyl or optionally substituted phenoxy, or two adjacent radicals, together with the adjacent C atoms, form an optionally substituted fused-on benzene ring or heterocyclic five- or six-membered rings which contain oxygen and are mono- or polysubstituted by fluorine. In formula I either one of the radicals $R^1$ must represent fluoroalkoxy or fluoroalkylmercapto or two adjacent radicals must form, together with the adjacent C atoms, heterocyclic five- or six-membered rings which contain oxygen and are mono- or polysubstituted by fluorine,

$R^2$ represents hydrogen, $C_{1-4}$-alkyl, cyano or ethynyl,

$R^3$ represents radicals of the formula

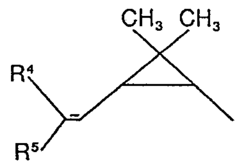

or

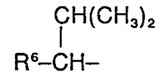

$R^4$ and $R^5$ represent chlorine or bromine, and

$R^6$ represent phenyl which is optionally substituted by halogen, alkyl, alkylthio or alkoxy, each with 1–4 C atoms, nitro or methylenedioxy, characterised in that

a) carbonyl halides of the formula II

$$Hal–CO–R^3 \qquad (II)$$

in which

$R^3$ has the meaning given (above), and

Hal represents halogen, preferably chlorine, are reacted with benzyl alcohols of the formula III

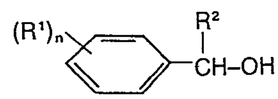

(III)

in which

$R^1$, n and $R^2$ have the meaning given above, if appropriate in the presence of an acid acceptor and if appropriate in the presence of a solvent, or

b) carboxylic acid salts of the formula IV

$$MeC–CO–R^3 \qquad (IV)$$

in which

Me denotes K or Na and

$R^3$ has the meaning given above,

are reacted with benzyl halides of the formula V

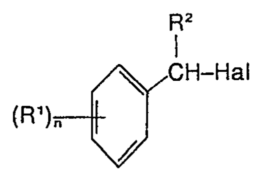

(V)

in which

$R^1$, n and $R^2$ have the meaning given above and

Hal denotes chlorine or bromine,

if appropriate in the presence of a solvent and if appropriate in the presence of a quaternary ammonium salt.

3. Benzyl alcohols of the formula IIIa

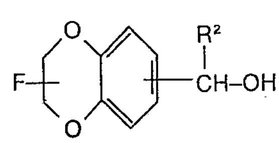

(IIIa)

in which

$R^2$ has the meaning given in Claim 1 and wherein the remaining positions in the benzene ring can be substituted by halogen.

4. Process for the preparation of the benzyl alcohols of the formula IIIa according to Claim 3, characterised in that

a) aldehydes of the formula VIa

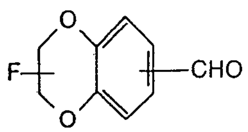

(VIa)

in which
the remaining positions in the benzene ring can be substituted by halogen,
in the case where $R^2$ in the benzyl alcohol represents hydrogen, are reduced, or, in the case where $R^2$ in the benzyl alcohol represents CN, are reacted with HCN, or, in the case where $R^2$ in the benzyl alcohol represents $C_{1-4}$-alkyl or ethynyl, are reacted with a Grignard compound of the formula VII

$R^2$-Mg-Hal (VII)

in which
$R^2$ represents $C_{1-4}$-alkyl or ethynyl, or
b) in that benzylamines of the formula VIIIa

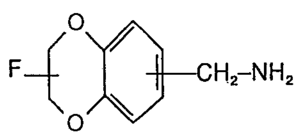

(VIIIa)

in which
the remaining positions in the benzene ring can be substituted by halogen,
are reacted with sodium nitrite or potassium nitrite in the presence of an acid or
c) in that a benzyl halide of the formula Va

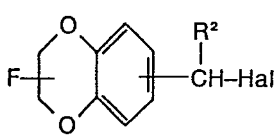

(Va)

in which
$R^2$ has the meaning given in Claim 1,
Hal represents halogen and the remaining positions in the benzene ring can be substituted by halogen,
is saponified with aqueous bases.

5. Benzyl halides of the formula Va

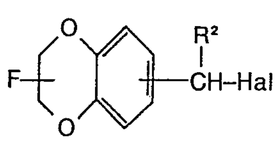

(Va)

in which
$R^2$ has the meaning given in Claim 1,
Hal represents halogen and the remaining positions in the benzene ring can be substituted by halogen.

6. Process for the preparation of the benzyl halides of the formula Va according to Claim 5, characterised in that compounds of the formula IXa

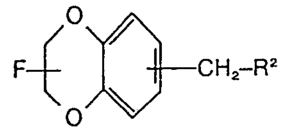

(IXa)

in which
$R^2$ has the meaning given in Claim 1 and
the remaining positions in the benzene ring can be substituted by halogen,
are halogenated in the side chain in a manner known per se.

7. Aldehydes of the formula VIa

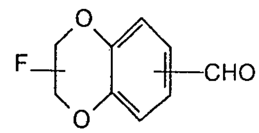

(VIa)

in which
the remaining positions in the benzene ring can be substituted by halogen.

8. Process for the preparation of the aldehydes of the formula VIa according to Claim 7, characterised in that in compounds of the formula XIa

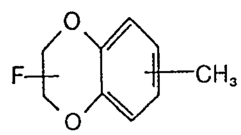

(XIa)

in which
the remaining positions in the benzene ring can be substituted by halogen,
the $CH_3$ group is halogenated, in a manner known per se, to give the $-CH\ Hal_2$ group which is then saponified, in a customary manner, to give the aldehyde of the formula VI.

9. Benzylamines of the formula VIIIa

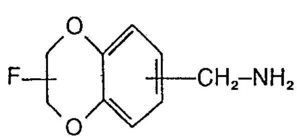

(VIIIa)

in which
the remaining positions in the benzene ring can be substituted by halogen.

10. Process for the preparation of the benzylamines of the formula VIIIa according to Claim 9, characterised in that a compound of the formula XIIa

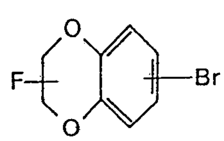

(XIIa)

in which
the remaining positions in the benzene ring can be substituted by halogen,
is reacted in a first stage with a cyanide salt, and the nitrile thus obtained, of the formula XIIIa

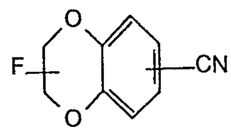

(XIIIa)

in which
the remaining positions in the benzene ring can be substituted by halogen,
is hydrogenated in a manner known per se.

11. Insecticides, characterised by a content of at least one benzyl ester of the formula I according to Claim 1.

12. Use of benzyl esters of the formula I according to Claim 1 for combating insects.

**Revendications**

1. Esters benzyliques de formule I

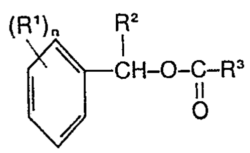

(I)

dans laquelle
n représente 1–5, et
$R^1$ représente des radicaux identiques ou différents choisis parmi le groupe comprenant un atome d'hydrogène, un groupe alkyle en $C_1$–$C_6$, un groupe halogénalkyle en $C_1$–$C_6$ contenant 3 à 6 atomes d'halogènes, un groupe alcoxy en $C_{1-6}$, un groupe halogénalcoxy en $C_1$–$C_6$ contenant 1 à 6 atomes d'halogènes, un groupe alkyl(en $C_1$–$C_6$)mercapto, un groupe halogénalkyl(en $C_1$–$C_6$)mercapto contenant 1 à 6 atomes d'halogènes, un atome d'halogène, un groupe phényle éventuellement substitué, ainsi qu'un groupe phénoxy éventuellement substitué, ou deux radicaux voisins, ensemble avec les atomes de carbone adjacents, forment un noyau benzène condensé éventuellement substitué ou des noyaux hétérocycliques pentagonaux ou hexagonaux contenant de l'oxygène et substitués une ou plusieurs fois par un atome de fluor; en formule I, un des radicaux $R^1$ doit représenter un groupe fluoralcoxy ou un groupe fluoralkylmercapto ou deux radicaux voisins doivent former, ensemble avec les atomes de carbone adjacents, des noyaux hétérocycliques pentagonaux ou hexagonaux contenant de l'oxygène et substitués une ou plusieurs fois par un atome de fluor,
$R^2$ représente un atome d'hydrogène, un groupe alkyle en $C_1$–$C_4$, un groupe cyano ou un groupe éthinyle,
$R^3$ représente les radicaux répondant aux formules:

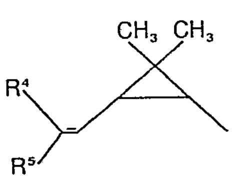

ou

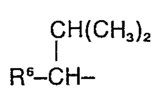

$R^4$ et $R^5$ représentent chacun un atome de chlore ou un atome de brome,
$R^6$ représente un groupe phényle éventuellement substitué par un atome d'halogène, par un groupe alkyle, alkylthio ou alcoxy contenant chacun 1 à 4 atomes de carbone, par un groupe nitro ou par un groupe méthylène-dioxy.

2. Procédé de préparation des esters benzyliques de formule I:

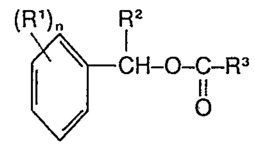

(I)

dans laquelle
n représente 1–5, et
$R^1$ représente des radicaux identiques ou différents choisis parmi le groupe comprenant un atome d'hydrogène, un groupe alkyle en $C_1$–$C_6$, un groupe halogénalkyle en $C_1$–$C_6$ contenant 3 à 6 atomes d'halogènes, un groupe alcoxy en $C_1$–$C_6$, un groupe halogénalcoxy en $C_1$–$C_6$ contenant 1 à 6 atomes d'halogènes, un groupe alkyl(en $C_1$–$C_6$)mercapto, un groupe halogénalkyl(en $C_1$–$C_6$)mercapto contenant 1 à 6 atomes d'halogènes, un atome d'halogène, un groupe phényle éventuellement substitué, ainsi qu'un groupe phénoxy éventuellement substitué, ou deux radicaux voisins, ensemble avec les atomes de carbone adjacents, forment un noyau benzène condensé éventuellement substitué ou des noyaux hétérocycliques pentagonaux ou hexagonaux contenant de l'oxygène et substitués une ou plusieurs fois par un atome de fluor; en formule I, un des radicaux $R^1$ doit représenter un groupe fluoralcoxy ou un groupe fluoralkylmercapto ou deux radicaux voisins doivent former, ensemble avec les atomes de carbone adjacents, des noyaux hétérocycliques pentagonaux ou hexagonaux contenant de l'oxygène et substitués une ou plusieurs fois par un atome de fluor,
$R^2$ représente un atome d'hydrogène, un groupe alkyle en $C_1$–$C_4$, un groupe cyano ou un groupe éthinyle,
$R^3$ représente les radicaux répondant aux formules:

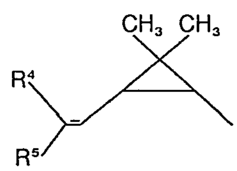

ou

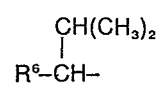

$R^4$ et $R^5$ représentent chacun un atome de chlore ou un atome de brome,
$R^6$ représente un groupe phényle éventuellement substitué par un atome d'halogène, par un groupe alkyle, alkylthio ou alcoxy contenant chacun 1 à 4 atomes de carbone, par un groupe nitro ou par un groupe méthylène-dioxy,
caractérisé en ce que:
a) on fait réagir des halogénures de carbonyle de formule II:

$$Hal\text{–}CO\text{–}R^3 \qquad (II)$$

dans laquelle

$R^3$ a la signification indiquée (ci-dessus), et

Hal représente un atome d'halogène, de préférence, un atome de chlore,

avec des alcools benzyliques de formule III:

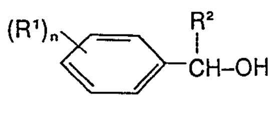

(III)

dans laquelle

$R^1$, n et $R^2$ ont les significations indiquées ci-dessus, éventuellement en présence d'un accepteur d'acide et éventuellement en présence d'un solvant, ou

b) on fait réagir des sels d'acides carboxyliques de formule IV:

$$MeO{-}CO{-}R^3 \qquad (IV)$$

dans laquelle

Me représente K ou Na, et

$R^3$ a la signification indiquée ci-dessus,

avec des halogénures de benzyle de formule V:

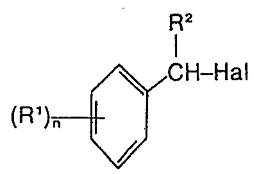

(V)

dans laquelle

$R^1$, n et $R^2$ ont les significations indiquées ci-dessus, et

Hal représente un atome de chlore ou un atome de brome, éventuellement en présence d'un solvant et éventuellement en présence d'un sel d'ammonium quaternaire.

3. Alcools benzyliques de formule IIIa:

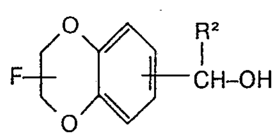

(IIIa)

dans laquelle

$R^2$ a la signification indiquée dans la revendication 1, les autres positions du noyau benzène pouvant être substituées par des atomes d'halogènes.

4. Procédé de préparation des alcools benzyliques de formule IIIa selon la revendication 3, caractérisé en ce que:

a) lorsque, dans l'alcool benzylique, $R^2$ représente l'hydrogène, on réduit des aldéhydes de formule VIa:

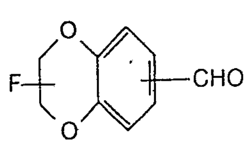

(VIa)

dans laquelle

les autres positions du noyau benzène peuvent être substituées par des atomes d'halogènes,

ou lorsque, dans l'alcool benzylique, $R^2$ représente CN, on les fait réagir avec HCN,

ou encore, lorsque, dans l'alcool benzylique, $R^2$ représente un groupe alkyle en $C_1$–$C_4$ ou un groupe éthinyle, on les fait réagir avec un composé de Grignard de formule VII:

$$R^2{-}Mg{-}Hal \qquad (VII)$$

dans laquelle

$R^2$ représente un groupe alkyle en $C_1$–$C_4$ ou un groupe éthinyle, ou

b) on fait réagir des benzylamines de formule VIIIa:

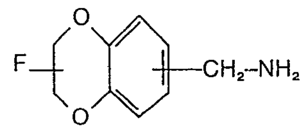

(VIIIa)

dans laquelle

les autres positions du noyau benzène peuvent être substituées par des atomes d'halogènes,

avec du nitrite de sodium ou du nitrite de potassium en présence d'un acide, ou

c) on saponifie, avec des bases aqueuses, un halogénure de benzyle de formule Va:

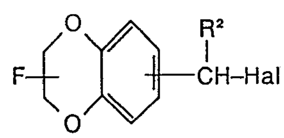

(Va)

dans laquelle

$R^2$ a la signification indiquée dans la revendication 1,

Hal représente un atome d'halogène et les autres positions du noyau benzène peuvent être substituées par des atomes d'halogènes.

5. Halogénures de benzyle de formule Va:

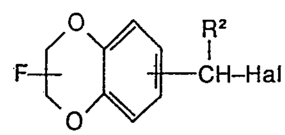

(Va)

dans laquelle

$R^2$ a la signification indiquée dans la revendication 1,

Hal représente un atome d'halogène et les autres positions du noyau benzène peuvent être substituées par des atomes d'halogènes.

6. Procédé de préparation des halogénures de benzyle de formule Va selon la revendication 5, caractérisé en ce qu'on soumet, à une halogénation dans la chaîne latérale et de façon connue en soi, des composés de formule IXa:

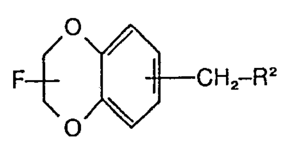

(IXa)

dans laquelle

$R^2$ a la signification indiquée dans la revendication 1 et les autres positions du noyau benzène peuvent être substituées par des atomes d'halogènes.

7. Aldéhydes de formule VIa:

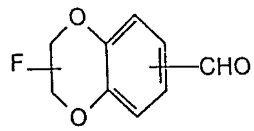

(VIa)

dans laquelle

les autres positions du noyau benzène peuvent être substituées par des atomes d'halogènes.

8. Procédé de préparation des aldéhydes de formule VIa selon la revendication 7, caractérisé en ce que, dans des composés de formule XIa:

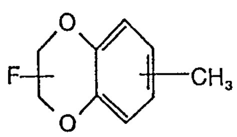

(XIa)

dans laquelle

les autres positions du noyau benzène peuvent être substituées par des atomes d'halogènes,

on soumet le groupe $CH_3$ à une halogénation de façon connue en soi en un groupe $-CH\,Hal_2-$ et on saponifie ensuite de la manière habituelle en un aldéhyde de formule VI.

9. Benzylamines de formule VIIIa:

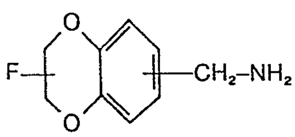

(VIIIa)

dans laquelle

les autres positions du noyau benzène peuvent être substituées par des atomes d'halogènes.

10. Procédé de préparation des benzylamines de formule VIIIa selon la revendication 9, caractérisé en ce que, dans une première étape, on fait réagir un composé de formule XIIa:

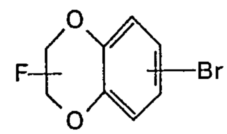

(XIIa)

dans laquelle

les autres positions du noyau benzène peuvent être substituées par des atomes d'halogènes,

avec un sel cyanure et l'on soumet le nitrile ainsi obtenu de formule XIIIa:

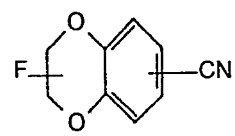

(XIIIa)

dans laquelle

les autres positions du noyau benzène peuvent être substituées par des atomes d'halogènes,

à une hydrogénation de façon connue en soi.

11. Insecticides, caractérisés en ce quils contiennent au moins un ester benzylique de formule I selon la revendication 1.

12. Utilisation d'esters benzyliques de formule I selon la revendication 1, pour combattre les insectes.